# FASCICULE DE BREVET EUROPEEN

(11) **EP 1 484 009 B1**
(45) Date de publication et mention de la délivrance du brevet: **15.02.2006**
(21) Numéro de dépôt: 03012707.0
(22) Date de dépôt: 04.06.2003
(51) Int. Cl.: A61B 5/024, A61B 5/00

(54) **Instrument portable muni d'un dispositif optique de mesure d'une grandeur physiologique et de moyens pour l'emission et/ou la reception de donnees**
Portables Instrument mit optischer Messeinheit zur Messung einer physiologischen Grösse und Mitteln zur Übertragung und/oder zum Empfang von Daten
Portable instrument with optical measuring means for measuring a physiological value and means for sending and/or receiving data

(43) Date de publication de la demande: 08.12.2004
(73) Titulaire: ETA SA Manufacture Horlogère Suisse, 2540 Grenchen (CH)
(72) Inventeur: Blondeau, Fabien, 2525 Le Landeron (CH); Karapatis, Nakis, 2520 La Neuveville (CH)
(74) Mandataire: Ravenel, Thierry Gérard Louis

(56) Documents cités:
- EP-A- 0 842 635
- EP-A- 1 297 784
- US-A- 5 776 056
- US-A- 5 810 736
- US-A1- 2002 165 436

## Description

### DOMAINE TECHNIQUE

La présente invention concerne de manière générale un instrument portable comprenant un dispositif optique pour la mesure d'une grandeur physiologique, notamment du rythme cardiaque, et des moyens pour l'émission et/ou la réception de données, ce dispositif optique comprenant au moins une source lumineuse pour soumettre une portion d'un tissu organique à une émission lumineuse et au moins un photorécepteur pour détecter l'intensité de l'émission lumineuse après propagation dans le tissu organique.

### ARRIÈRE-PLAN TECHNOLOGIQUE

De tels instruments portables de mesure sont déjà connus. Ces dispositifs portables sont notamment employés pour permettre la détection, par voie optique, du rythme cardiaque et/ou du taux d'oxygène dans le sang d'un patient. On les trouve sous des formes variées allant de pinces destinées à être placées sur une zone du corps humain (typiquement sur l'extrémité d'un doigt, sur le lobe de l'oreille ou sur toute autre extrémité du corps humain suffisamment irriguée par le sang) à des dispositifs portés au poignet présentant une allure analogue à celle d'une montre-bracelet.

Dans le cadre d'une application à la mesure du rythme cardiaque, le dispositif optique est utilisé pour produire une illumination adéquate d'une partie du tissu organique (typiquement la peau) et comporte un ou plusieurs photorécepteurs pour détecter l'intensité de l'émission lumineuse produite par le dispositif optique après propagation dans le tissu organique. Les variations de pulsation du flux sanguin induisent une variation de l'absorption de l'émission lumineuse produite par le dispositif optique, variation d'absorption dont la fréquence correspond essentiellement à la fréquence des pulsations cardiaques. Une détection de l'intensité de l'émission lumineuse après propagation dans le tissu organique accompagnée d'un traitement adéquat du ou des signaux de mesure permet d'extraire une indication du rythme cardiaque. Les dispositifs optiques communément employés pour ce type d'application sont relativement simples et consistent typiquement en une ou plusieurs sources lumineuses quasi-ponctuelles, typiquement des diodes électroluminescentes ou LED ("light-emitting diode") émettant dans une gamme de longueurs d'onde déterminée, associées à un ou plusieurs photorécepteurs, typiquement des photodiodes.

Outre la fonction de mesure de la grandeur physiologique désirée, les instruments portables équipés de dispositifs optiques du type susmentionné sont par ailleurs communément équipés de moyens pour émettre et/ou recevoir des données. En particulier, des moyens d'émission sont typiquement prévus pour permettre le déchargement sur un terminal externe de données mesurées et mémorisées dans l'instrument portable durant des périodes d'activité, par exemple lors d'une activité physique ou au cours d'un diagnostic de santé. D'autre part, des moyens de réception peuvent être prévus pour charger des données de configuration de l'instrument portable, par exemple des valeurs limites de la grandeur physiologique mesurée telles des valeurs minimale et maximale du rythme cardiaque entre lesquelles l'utilisateur désire maintenir son rythme cardiaque. Les données émises depuis l'instrument portable ou reçues par l'instrument portable peuvent ou non être liées à la grandeur physiologique mesurée.

L'émission et/ou la réception de données peut communément être opérée par liaison directe par câble ou préférablement grâce à des moyens de communication sans fils qui peuvent par exemple être du type acoustique, optique, inductif ou radio-fréquence.

Les documents US 4,674,743, EP 0 842 635 et US 5,776,056 décrivent divers exemples d'instruments portables munis d'un dispositif optique du type susmentionné pour la mesure d'une grandeur physiologique ainsi que de moyens de communication de données par voie optique. Ces documents décrivent toutefois des solutions employant des dispositifs optiques distincts et divulguent les caractéristiques du préambule de la revendication 1.

Comme mentionné, d'autres solutions connues font appel à des moyens de communication du type acoustique, inductif ou radio-fréquence. On peut citer par exemple les documents EP 0 940 119, US 5,810,736, US 5,622,180 ou encore WO 99/41647 et EP 1 101 439.

Toutes les solutions antérieures susmentionnées, y compris les solutions employant des moyens optiques de communication, présentent l'inconvénient de nécessiter des composants additionnels spécifiques qui pèsent sur les coûts de fabrication de l'instrument portable et requièrent inévitablement de la place pour être incorporé. Ces solutions ne sont donc pas optimales en termes d'encombrement et de coûts de fabrication.

Un but général de la présente invention est donc de proposer un instrument portable du type susmentionné qui permette à la fois une réduction des coûts et une réduction de l'encombrement de l'instrument portable par rapport aux solutions de l'art antérieur.

### RÉSUMÉ DE L'INVENTION

La présente invention a ainsi pour objet un instrument portable dont les caractéristiques sont énoncées dans la revendication 1.

Des modes de réalisation avantageux de la présente invention font l'objet des revendications dépendantes.

Selon la solution proposée, il est ainsi proposé d'utiliser le dispositif optique normalement employé comme moyen de mesure de la grandeur physiologique comme moyen d'émission et/ou de réception de données. Plus particulièrement, une source lumineuse au moins du dispositif optique est utilisée pour émettre des données à destination d'une unité externe, et/ou un photorécepteur au moins du dispositif optique est utilisé pour recevoir des données transmises depuis l'unité externe.

En fonctionnement, le dispositif optique est avantageusement commuté entre une phase de mesure durant laquelle il opère comme moyen de mesure de la grandeur physiologique et une phase de communication durant laquelle il opère comme moyen d'émission et/ou de réception de données, l'instrument portable comportant en outre des moyens de mémorisation pour stocker, durant la phase de mesure, des données relatives à l'évolution au cours du temps de ladite grandeur physiologique avant leur transmission, durant la phase de communication, vers l'unité externe.

Selon des modes de réalisation particuliers, il est par ailleurs proposé des moyens pour activer automatiquement la fonction de mesure ou la fonction d'émission/réception de données du dispositif optique.

La solution proposée présente ainsi l'avantage d'une utilisation optimale de composants déjà présents dans l'instrument portable réduisant ainsi les coûts de fabrication et favorisant une meilleure utilisation de l'espace disponible dans l'instrument.

### BRÈVE DESCRIPTION DES DESSINS

D'autres caractéristiques et avantages de la présente invention apparaîtront plus clairement à la lecture de la description détaillée qui suit de divers modes de réalisation de l'invention donnés uniquement à titre d'exemples non limitatifs et illustrés par les dessins annexés où :

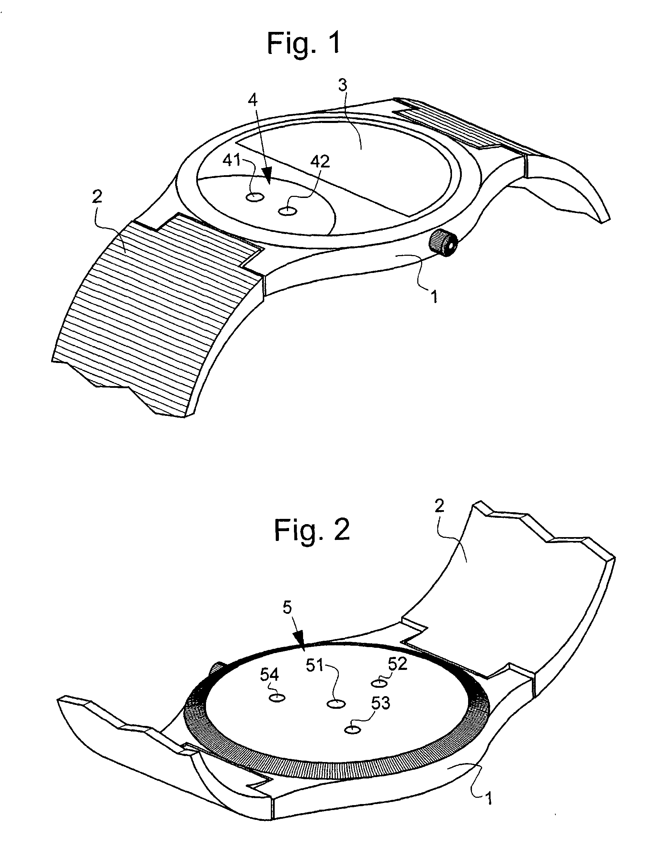

- la figure 1 montre une vue en perspective côté affichage d'un instrument portable de mesure d'une grandeur physiologique selon un premier exemple de réalisation se présentant sous une forme analogue à celle d'une montre-bracelet et comprenant un dispositif optique agencé sur la face avant de l'instrument ;
- la figure 2 montre une vue en perspective côté fond d'un instrument portable de mesure d'une grandeur physiologique selon un second exemple de réalisation se présentant également sous une forme analogue à celle d'une montre-bracelet et comprenant un dispositif optique agencé dans le fond de l'instrument ;

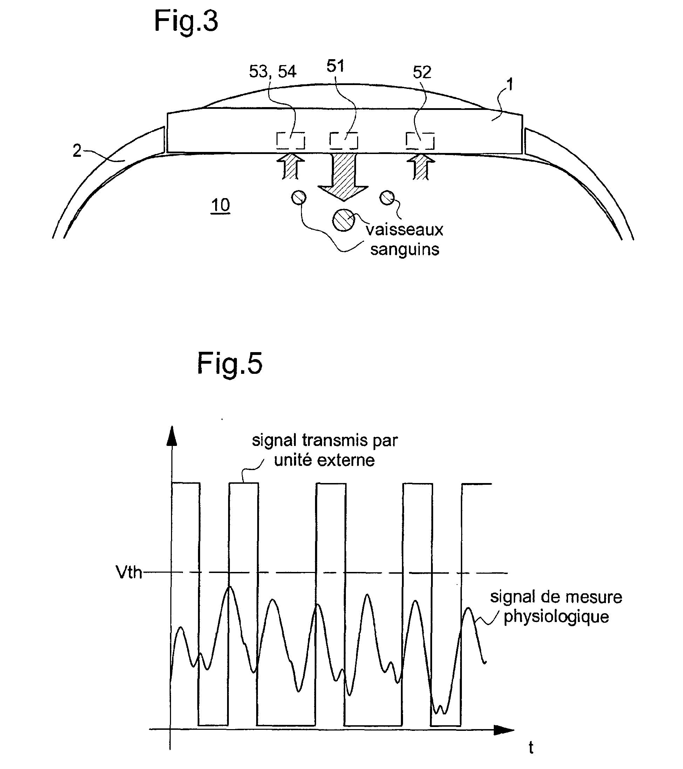

- la figure 3 est une vue en coupe de l'instrument de la figure 2 prise selon la direction longitudinale du bracelet lorsque cet instrument est porté au poignet pour entreprendre la mesure de la grandeur physiologique ;

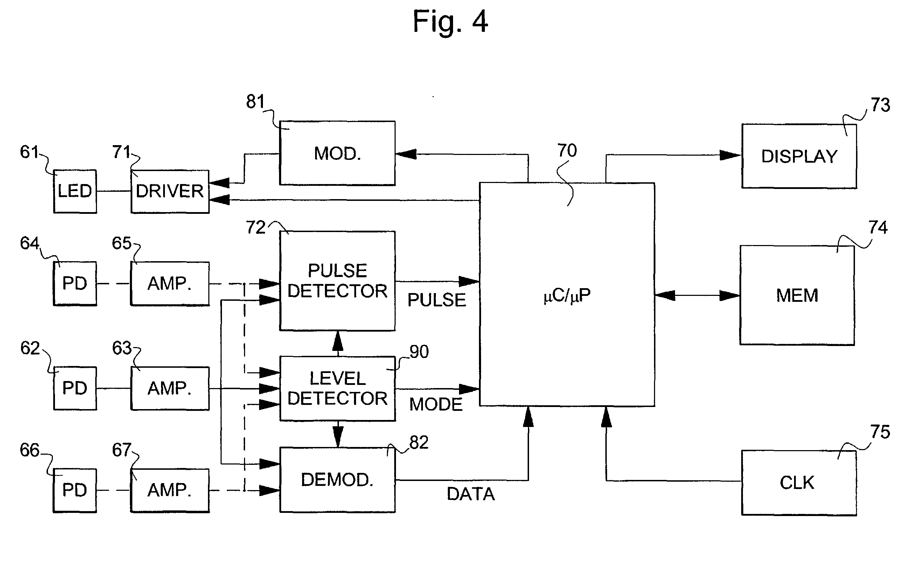

- la figure 4 est un schéma bloc illustrant divers composants de l'instrument portable selon un exemple de réalisation ; et
- la figure 5 est un diagramme illustrant schématiquement comment il est envisageable de différencier des signaux optiques transmis par une unité externe des signaux optiques captés lors d'une mesure de la grandeur physiologique, ceci dans l'optique d'activer sélectivement le mode de fonctionnement adéquat de l'instrument portable.

### DESCRIPTION DES MODES DE RÉALISATION PRÉFÉRÉS

Les divers modes de réalisation qui vont maintenant être présentés sont donnés à titre purement illustratif et non limitatif. En particulier, il convient d'insister sur le fait que les modes de réalisation qui vont être présentés peuvent avantageusement mais pas uniquement être implémentés dans un instrument destiné à être porté au poignet. D'autres applications portables de ces solutions sont parfaitement envisageables.

Les figures 1 et 2 montrent à titre purement illustratif et non limitatif deux exemples de réalisation d'un instrument portable de mesure d'une grandeur physiologique qui se présentent tous deux sous une forme analogue à celle d'une montre-bracelet. Tous deux comportent ainsi une boîte 1 formant carrure dans cet exemple et un bracelet 2 attaché de manière conventionnelle à la boîte 1. Un dispositif d'affichage 3 est par ailleurs disposé sur la face avant de l'instrument, ce dispositif d'affichage 3 n'apparaissant que sur l'exemple de la figure 1. La différence entre les deux exemples des figures 1 et 2 réside essentiellement dans l'emplacement et la configuration du dispositif optique (désigné globalement par la référence numérique 4, resp. 5) employé pour la mesure de la grandeur physiologique désirée (par exemple le rythme cardiaque ou le taux d'oxygène dans le sang comme déjà mentionné).

Dans l'exemple de la figure 1, le dispositif optique 4 est placé sur la face avant de l'instrument portable au voisinage du dispositif d'affichage 3. Le dispositif optique 4 comporte ici une unique source lumineuse 41 coopérant avec un unique photorécepteur 42. Comme déjà mentionné, la source lumineuse 41 est typiquement une diode électroluminescente (LED) émettant dans une gamme de longueurs d'onde déterminée (par exemple dans l'infrarouge ou toute autre gramme de longueurs d'onde adéquate) et le photorécepteur 42 peut être une photodiode, un phototransistor ou tout autre récepteur optique adéquat ayant une réponse adaptée à la gamme de longueurs d'onde de la source lumineuse 41.

Selon l'exemple de la figure 1, on comprendra que la mesure de la grandeur physiologique désirée est par exemple opérée par le placement d'un doigt sur la face avant de l'instrument portable en regard du dispositif optique 4.

Dans l'exemple de la figure 2, le dispositif optique, désigné 5, est placé dans le fond de l'instrument. A la différence de l'exemple de la figure 1, le dispositif optique 5 comporte, outre une source lumineuse 51 placée essentiellement dans une région centrale du fond de l'instrument portable, trois photorécepteurs 52, 53 et 54 placés symétriquement autour de la source lumineuse centrale 51. L'utilisation de plusieurs photorécepteurs agencés autour d'une ou plusieurs sources lumineuses est généralement préférée à la solution de la figure 1, ceci essentiellement dans l'optique d'assurer une meilleure fiabilité de mesure. Sur ce dernier point, pour obtenir de plus amples informations sur un système de mesure optique du rythme cardiaque employant au moins deux canaux de détection, on pourra se référer au document EP 1 297 784 qui montre par ailleurs un exemple de réalisation d'un instrument portable analogue à celui de la figure 2.

La figure 3 est une vue en coupe de l'instrument portable de la figure 2 prise dans la direction longitudinale du bracelet lorsque ce dernier est porté au poignet (désigné par la référence numérique 10 dans la figure). Au contraire de l'exemple de la figure 1, l'instrument portable et son dispositif optique 5 est ici amené en permanence au contact du tissu organique de l'utilisateur lorsque l'instrument est porté. L'émission lumineuse produite par la source 51 est agencée pour pénétrer suffisamment profondément dans le tissu organique pour être modulée par le flux sanguin irriguant le tissu organique illuminé. L'émission lumineuse modulée est détectée après réflexion par les photorécepteurs 52 à 54 du dispositif optique.

La figure 4 présente un schéma bloc illustrant les composants constitutifs principaux de l'instrument portable selon un mode de réalisation de l'invention. Comme illustré, l'instrument comporte une source lumineuse 61 (par exemple une diode électroluminescente (LED) ou tout autre dispositif adéquat) couplée à un circuit de commande 71 dont le fonctionnement est contrôlé par une unité centrale de traitement 70, tel un microprocesseur ou microcontrôleur. Cette unité centrale 70 est par ailleurs interfacée avec un dispositif d'affichage 73 (de type analogique et/ou digital), des moyens de mémorisation 74 (RAM, ROM, EEPROM, FLASH ou analogues) et une horloge système 75 assurant le cadencement adéquat du fonctionnement de l'unité centrale 70 et de ses composants périphériques. Cette horloge système 75 peut par ailleurs assurer les fonctions horaires classiques d'un garde-temps.

L'unité centrale de traitement 70 est encore couplée à un circuit 72 dédié à la détection et la mesure de la grandeur physiologique désirée (notamment le rythme cardiaque), les fonctions de ce circuit pouvant néanmoins être intégrées avec celles de l'unité centrale de traitement. Les fonctions de ce circuit ont déjà brièvement été abordées et visent essentiellement à extraire les informations relatives à la grandeur physiologique à partir des signaux optiques détectés par le ou les photorécepteurs associés. Dans le cas d'espèce, un premier photorécepteur 62 est couplé au circuit de détection 72 via un moyen d'amplification et, le cas échéant, de filtrage 63. Les informations relatives à la grandeur physiologique désirée sont transmises à l'unité centrale 72, notamment dans le but d'être affichées sur le dispositif 73 et/ou, préférablement, stockées dans les moyens de mémorisation 74 pour une consultation ultérieure.

On ne s'étendra pas ici sur le procédé de mesure de la grandeur physiologique car cette question ne concerne pas directement l'objet de la présente invention. D'autre part, diverses solutions existent pour opérer cette mesure. Une solution particulièrement performante pour la mesure du rythme cardiaque est amplement détaillée dans le document EP 1 297 784 déjà mentionné.

Dans l'exemple de réalisation de la figure 4, dans l'optique de pouvoir transmettre et/ou recevoir des données au moyen de son dispositif optique, l'instrument portable comporte en outre, d'une part, un moyen de modulation 81 couplé entre le circuit de commande 71 de la source lumineuse et l'unité centrale 70, et, d'autre part, un moyen de démodulation 82 couplé entre le moyen d'amplification 63 associé au photorécepteur 62 et l'unité centrale 70.

On comprendra que le moyen de modulation 81 a pour but de contrôler le circuit de commande 71 de manière à produire un signal optique modulé au moyen de la source lumineuse associée 61. De même, on comprendra que le moyen de démodulation 82 à pour but inverse de décoder un signal lumineux modulé capté par le photorécepteur associé 62. Le type de modulation utilisée pour la transmission et la réception des données peut être quelconque. Il peut classiquement s'agir d'une modulation d'amplitude, de phase, de fréquence ou de code. D'autre part, des modulations distinctes pourraient être adoptées pour transmettre et recevoir les données de manière à clairement identifier s'il s'agit d'une émission entrante ou sortante. En tous les cas, les signaux optiques sont modulés sur la base des données à transmettre.

Dans la figure 4, on notera que l'instrument portable peut comporter plus d'une source lumineuse et/ou plus d'un photorécepteur. En particulier, comme schématisé par les traits interrompus dans la figure 4, un deuxième photorécepteur 64 et un moyen d'amplification associé 65 pourraient être couplés au détecteur 72, ceux-ci n'étant pas nécessairement couplés au moyen de démodulation 82. De même, un troisième photorécepteur 66 et un moyen d'amplification associé 67 pourraient être couplés au moyen de démodulation 82 sans toutefois l'être au détecteur 72. Ce troisième photorécepteur ne participerait ainsi pas à la détection de la grandeur physiologique désirée mais uniquement à la réception de données.

D'une manière générale, dans le cadre de la présente invention, on notera qu'il suffira déjà qu'au moins une source lumineuse ou un photorécepteur du dispositif optique, normalement utilisé dans le cadre de la mesure de la grandeur physiologique désirée, soit par ailleurs utilisé pour transmettre ou recevoir, respectivement, des données. On peut en effet déjà espérer obtenir un avantage en termes de réduction des coûts de fabrication et de simplification de la construction en utilisant qu'une source lumineuse ou qu'un photorécepteur pour remplir les deux fonctions. On notera en outre qu'il pourra être préférable d'utiliser un photorécepteur spécifique dédié uniquement à la mesure de la grandeur physiologique et un photorécepteur additionnel pour opérer la réception de données, les contraintes en termes de sensibilité pouvant le justifier.

Dans le cadre de la présente invention, il sera par ailleurs avantageux de munir l'instrument de moyens de détection pour activer automatiquement et sélectivement la fonction de mesure de grandeur physiologique ou d'émission/réception de données du dispositif optique.

Au titre de premier exemple, l'instrument portable pourrait ainsi comporter des premiers moyens de détection pour activer automatiquement la fonction de mesure de la grandeur physiologique du dispositif optique lorsque ce dernier est amené au contact du tissu organique. Une solution avantageuse pourrait consister à déterminer si le dispositif optique est au contact du tissu organique sur la base de l'intensité de l'émission lumineuse détectée par au moins un photorécepteur.

Au titre de second exemple, l'instrument portable pourrait également comporter des seconds moyens de détection pour activer automatiquement la fonction d'émission et/ou de réception de données du dispositif optique lorsque des données sont émises à l'intention de l'instrument portable. Une solution avantageuse pourrait également consister à détecter si des données sont émises à l'intention de l'instrument portable sur la base de l'intensité de l'émission lumineuse détectée par au moins un photorécepteur.

En se référant à nouveau à la figure 4, il sera ainsi avantageux de pourvoir en outre l'instrument portable de moyens 90 pour détecter le niveau d'intensité de l'émission lumineuse captée par un voire plusieurs photorécepteurs. Il serait en pratique envisageable de différencier un signal optique transmis par une unité externe de ou des signaux optiques typiquement captés par ces mêmes photorécepteurs lorsque l'instrument portable opère en mode de mesure, l'intensité du signal optique produit par une unité externe pouvant être ajusté pour présenter un niveau d'intensité supérieur au niveau moyen des signaux optiques lors d'une mesure de grandeur physiologique.

La figure 5 illustre schématiquement cette possibilité par un diagramme d'évolution dans le temps où sont reportés à titre d'exemple un tracé représentatif d'une mesure optique du rythme cardiaque et un tracé représentatif d'un signal modulé transmis par une unité externe, les intensités respectives de ces signaux étant choisies pour être sensiblement différentes. En imposant un seuil de détection Vth comme illustré, les deux types de signaux pourraient ainsi être différenciés et l'instrument portable commuté dans le mode adéquat.

Partant de ce principe, une activation sélective du détecteur 72 ou du démodulateur 82 pourrait être entreprise par les moyens 90. Le démodulateur 82 pourrait par ailleurs être utilisé pour décoder et détecter la présence d'un signal optique de commande prédéterminé transmis par l'unité externe. Ainsi, un premier signal optique de commande codé selon une séquence connue de l'instrument portable pourrait être transmis par l'unité externe pour aviser l'instrument portable que des données (par exemple de configuration) lui seront transmises. De même, un second signal optique de commande codé selon une autre séquence de code connue de l'instrument portable pourrait être transmis par l'unité externe pour requérir l'émission par l'instrument portable de données (par exemple des données relatives à l'évolution au cours du temps de la grandeur physiologique mesurée stockées dans les moyens de mémorisation 74).

Si une différentiation des signaux optiques basée sur l'intensité des signaux captés uniquement ne suffit pas, les moyens 90 pourraient alternativement être agencés pour différencier les signaux sur la base de caractéristiques de modulation distinctives du signal optique transmis par l'unité externe, par exemple sur la base d'une analyse fréquentielle des signaux reçus. On comprendra que d'autres moyens équivalents peuvent encore être envisagés.

On comprendra enfin que diverses modifications et/ou améliorations évidentes pour l'homme du métier peuvent être apportées aux modes de réalisation décrits dans la présente description sans sortir du cadre de l'invention défini par les revendications annexées. En particulier, la présente invention n'est pas limitée uniquement à une utilisation dans une montre-bracelet mais s'applique à toute autre application portable, au poignet ou non.

## Revendications

1. Instrument portable comprenant un dispositif optique (4; 5; 6) pour la mesure d'une grandeur physiologique, notamment du rythme cardiaque, et des moyens pour l'émission et/ou la réception de données, ledit dispositif optique (4; 5; 6) comprenant au moins une source lumineuse (41; 51; 61) pour soumettre une portion d'un tissu organique (10) à une émission lumineuse et au moins un photorécepteur (42; 52, 53, 54; 62, 64, 66) pour détecter l'intensité de l'émission lumineuse après propagation dans ledit tissu organique,
**caractérisé en ce que** ledit dispositif optique forme (4; 5; 6) également lesdits moyens pour l'émission et/ou la réception de données, ladite au moins une source lumineuse (41; 51; 61) et/ou ledit au moins un photorécepteur (42; 52, 53, 54; 62, 64, 66) étant agencés pour respectivement émettre des données à destination d'une unité externe ou recevoir des données depuis ladite unité externe.

2. Instrument portable selon la revendication 1, **caractérisé en ce que** ledit dispositif optique (4; 5; 6) est commuté entre une phase de mesure durant laquelle il opère comme moyen de mesure de ladite grandeur physiologique et une phase de communication durant laquelle il opère comme moyen d'émission et/ou de réception de données,
l'instrument portable comportant en outre des moyens de mémorisation (74) pour stocker, durant ladite phase de mesure, des données relatives à l'évolution au cours du temps de ladite grandeur physiologique avant leur transmission, durant ladite phase de communication, vers ladite unité externe.

3. Instrument portable selon la revendication 1, **caractérisé en ce que** ledit dispositif optique (4; 5; 6) est agencé de sorte que, lorsque ledit instrument portable est porté, il est amené en contact permanent avec le tissu organique (10) pour opérer comme moyen de mesure de la grandeur physiologique, ledit dispositif optique étant agencé pour opérer comme moyen d'émission et/ou de réception de données lorsque ledit instrument portable n'est pas porté.

4. Instrument portable selon l'une quelconque des revendications 1 à 3, **caractérisé en ce qu'**il comporte des premiers moyens de détection (90) pour activer automatiquement la fonction de mesure de la grandeur physiologique dudit dispositif optique (4; 5; 6) lorsque ce dernier est amené au contact du tissu organique.

5. Instrument portable selon la revendication 4, **caractérisé en ce que** lesdits premiers moyens de détection (90) déterminent si ledit dispositif optique est au contact du tissu organique sur la base de l'intensité de l'émission lumineuse détectée par au moins un photorécepteur du dispositif optique.

6. Instrument portable selon l'une quelconque des revendications précédentes, **caractérisé en ce qu'**il comporte des seconds moyens de détection (90) pour activer automatiquement la fonction d'émission et/ou de réception de données dudit dispositif optique (4; 5; 6) lorsque des données sont émises à l'intention dudit instrument portable.

7. Instrument portable selon la revendication 6, **caractérisé en ce que** lesdits seconds moyens de détection (90) détectent si des données sont émises à l'intention de l'instrument portable sur la base de l'intensité de l'émission lumineuse détectée par au moins un photorécepteur du dispositif optique.

8. Instrument portable selon la revendication 6 ou 7, **caractérisé en ce que** la fonction d'émission et la fonction de réception de données dudit dispositif optique sont sélectionnées sur la base de signaux optiques de commande prédéterminés transmis par l'unité externe.

9. Utilisation d' un instrument portable selon l'une des revendications précédentes, dans lequel ledit dispositif optique est utilisé comme moyen d'émission et/ou de réception de données.

## Patentansprüche

1. Tragbares Instrument, das eine optische Vorrichtung (4; 5; 6) zum Messen einer physiologischen Größe, insbesondere des Herzrhythmus, sowie Mittel zum Senden und/oder Empfangen von Daten umfasst, wobei die optische Vorrichtung (4; 5; 6) wenigstens eine Lichtquelle (41; 51; 61), um einen Abschnitt eines organischen Gewebes (10) einer Lichtemission zu unterwerfen, und wenigstens einen Photoempfänger (42; 52, 53, 54; 62, 64, 66), um die Stärke der Lichtemission nach der Ausbreitung in dem organischen Gewebe zu erfassen, enthält,
**dadurch gekennzeichnet, dass** die optische Vorrichtung (4; 5; 6) außerdem die Mittel zum Senden und/oder Empfangen von Daten bildet, wobei die wenigstens eine Lichtquelle (41; 51; 61) und/oder der wenigstens eine Photoempfänger (42; 52, 53, 54; 62, 64, 66) so beschaffen sind, dass sie Daten zu einer externen Einheit senden bzw. Daten von dieser externen Einheit empfangen.

2. Tragbares Instrument nach Anspruch 1, **dadurch gekennzeichnet, dass** die optische Vorrichtung (4; 5; 6) zwischen einer Messphase, in der sie als Mittel zum Messen der physiologischen Größe arbeitet, und einer Kommunikationsphase, in der sie als Mittel zum Senden und/oder Empfangen von Daten arbeitet, umgeschaltet wird,
wobei das tragbare Instrument außerdem Speichermittel (74) umfasst, um in der Messphase Daten bezüglich der zeitlichen Entwicklung der physiologischen Größe zu speichern, bevor die Daten während der Kommunikationsphase an die externe Einheit übertragen werden.

3. Tragbares Instrument nach Anspruch 1, **dadurch gekennzeichnet, dass** die optische Vorrichtung (4; 5; 6) so beschaffen ist, dass sie, wenn das tragbare Instrument getragen wird, in einen ständigen Kontakt mit dem organischen Gewebe (10) gebracht wird, um als Mittel zum Messen der physiologischen Größe zu arbeiten, während die optische Vorrichtung so beschaffen ist, dass sie als Mittel zum Senden und/oder Empfangen von Daten arbeitet, wenn das tragbare Instrument nicht getragen wird.

4. Tragbares Instrument nach einem der Ansprüche 1 bis 3, **dadurch gekennzeichnet, dass** es erste Erfassungsmittel (90) umfasst, um die Funktion zum Messen der physiologischen Größe der optischen Vorrichtung (4; 5; 6) automatisch zu aktivieren, wenn diese letztere mit dem organischen Gewebe in Kontakt gebracht ist.

5. Tragbares Instrument nach Anspruch 4, **dadurch gekennzeichnet, dass** die ersten Erfassungsmittel (90) anhand der Stärke der Lichtstrahlung, die von dem wenigstens einen Photoempfänger der optischen Vorrichtung erfasst wird, feststellen, ob die optische Vorrichtung mit dem organischen Gewebe in Kontakt ist.

6. Tragbares Instrument nach einem der vorhergehenden Ansprüche, **dadurch gekennzeichnet, dass** es zweite Erfassungsmittel (90) umfasst, um die Funktion des Sendens und/oder Empfangens von Daten der optischen Vorrichtung (4; 5; 6) automatisch zu aktivieren, wenn die Daten zu dem tragbaren Instrument gesendet werden.

7. Tragbares Instrument nach Anspruch 6, **dadurch gekennzeichnet, dass** die zweiten Erfassungsmittel (90) anhand der Stärke der Lichtstrahlung, die von dem wenigstens einen Photoempfänger der optischen Vorrichtung erfasst wird, feststellen, ob Daten zu dem tragbaren Instrument gesendet werden.

8. Tragbares Instrument nach Anspruch 6 oder 7, **dadurch gekennzeichnet, dass** die Datensende- und die Datenempfangsfunktion der optischen Vorrichtung anhand von vorgegebenen optischen Steuersignalen, die von der externen Einheit übertragen werden, ausgewählt werden.

9. Verwendung eines tragbaren Instruments nach einem der vorhergehenden Ansprüche, in der die optische Vorrichtung als Mittel zum Senden und/oder Empfangen von Daten verwendet wird.

## Claims

1. Portable instrument including an optical device (4; 5; 6) for measuring a physiological quantity, particularly the heart rhythm, and means for emitting and/or receiving data, said optical device (4; 5; 6) including at least one light source (41; 51; 61) for subjecting a portion of an organic tissue (10) to a light emission and at least one photoreceptor (42; 52, 53, 54; 62, 64, 66) for detecting the intensity of the light emission after propagation in said organic tissue,
**characterised in that** said optical device (4; 5; 6) also forms said means for emitting and/or receiving data, said at least one light source (41; 51; 61) and/or said at least one photoreceptor (42; 52, 53, 54; 62, 64, 66) being arranged for respectively emitting data to an external unit or receiving data from said external unit.

2. Portable instrument according to claim 1, **characterised in that** said optical device (4; 5; 6) is switched between a measuring phase during which it operates as means for measuring said physiological quantity and a communication phase during which it operates as data emission and/or reception means,
the portable instrument further including memory means (74) for storing data, during said measuring phase, relating to the evolution over time of said physiological quantity prior to transmission of said data, during said communication phase, to said external unit.

3. Portable instrument according to claim 1, **characterised in that** said optical device (4; 5; 6) is arranged such that, when said portable instrument is being worn, it is brought into permanent contact with the organic tissue (10) in order to operate as means for measuring said physiological quantity, said optical device being arranged to operate as data emission and/or reception means when said portable instrument is not being worn.

4. Portable instrument according to any one of claims 1 to 3, **characterised in that** it includes first detection means (90) for automatically activating the physiological quantity measuring function of said optical device (4; 5; 6) when the latter is brought into contact with the organic tissue.

5. Portable instrument according to claim 4, **characterised in that** said first detection means (90) determine whether said optical device is in contact with the organic tissue on the basis of the intensity of the light emission detected by at least one photoreceptor of the optical device.

6. Portable instrument according to any one of the preceding claims, **characterised in that** it includes second detection means (90) for automatically detecting the data emission and/or reception function of said optical device (4; 5; 6) when data is emitted to said portable instrument.

7. Portable instrument according to claim 6, **characterised in that** said second detection means (90) detect whether data is emitted to the portable instrument on the basis of the intensity of the light emission detected by at least one photoreceptor of the optical device.

8. Portable instrument according to claim 6 or 7, **characterised in that** the data emission function and the data reception function of said optical device are selected on the basis of predetermined optical control signals transmitted by the external unit.

9. Use of a portable instrument according to any of the preceding claims, wherein said optical device is used as data emission and/or reception means.
